# EUROPEAN PATENT APPLICATION

(11) **EP 4 684 819 A1**
(43) Date of publication of application: **28.01.2026**
(21) Application number: 24306239.5
(22) Date of filing: 23.07.2024
(51) Int. Cl.: A61M 5/168, A61M 5/48

(54) **OCCLUSION DETECTOR FOR AN INJECTION DEVICE, AND INJECTION DEVICE INCLUDING THIS OCCLUSION DETECTOR**

(71) Applicant: Becton Dickinson France, 38800 Le Pont-de-Claix (FR)
(72) Inventor: DELOBELLE, Vincent, 38420 DOMENE (FR)
(74) Representative: Germain Maureau

(57) **Abstract**

The occlusion detector (1) is configured to detect occlusion during injection of a medical product into an injection site. The occlusion detector (1) includes: a fluid chamber (403) for allowing circulation of the medical product through the occlusion detector (1), a piston rod (5) configured for axially moving from an inactive position to an active position when a pressure exerted by the medical product in the fluid chamber (403) increases, and a mechanical indicator (6) configured for alerting a user when the piston rod (5) reaches the active position.

## Description

The present invention relates to an occlusion detection device for a medical device such as an injection device, and an injection device including the occlusion detection device.

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction away from the user's hand, and the "proximal direction" is to be understood as meaning the direction toward the user's hand.

Wearable injection devices are injection devices configured to be worn by a patient, and more specifically attached to the patient's skin, during a predetermined time period, usually ranging from several minutes or hours to several days. The wearable injection devices are configured to deliver a predetermined volume of medical product at a predetermined flow rate and at a predetermined time after activation.

Wearable injection devices usually include an occlusion detector for detecting occlusion of the fluid path during the medical product delivery process. Occlusions may be due to a tube or a catheter that may have bent for some reason. This partially or completely restricts the flow of the medical product that should be delivered to the patient. Occlusions may also result from a fluid pressure that somehow gets so high that the drug administration system is no longer powerful enough to circulate the medical product until the injection site.

It is known to use direct or indirect occlusion detection devices.

Direct occlusion detection devices typically include a pressure sensor for measuring the fluid pressure and indicating when a predetermined high pressure threshold is reached. Direct occlusion detectors may also include flowmeters for measuring the medical product flow and indicating when a predetermined low flow is measured inside the injection device.

Indirect occlusion detection devices usually include a deflectable membrane and a laser or a switch or a translation coder for measuring deflection of this membrane. When deflection of the membrane gets higher than a predetermined threshold, then occlusion is detected. Indirect occlusion detection devices may alternatively include a motor consumption intensity or a motor torque management system that can detect when a occlusion occurs on the basis of the motor consumption intensity level or the motor torque level.

When occlusion is detected, the user usually gets informed by means of electronic devices such as a LED, a buzzer or a shaker and so on.

However, all the occlusion detectors are based on electronic components which may require calibration and/or which may be expensive. Transfer function from measured variable to pressure can be complex to estimate and to make robust. Besides, electronic components are subject to some drawbacks such as electromagnetic compatibility, or power supply, etc. The recycling issue also make these occlusion detectors less sustainable.

There is therefore a need for an occlusion detector that alleviates some or all of the aforementioned drawbacks of the prior art. More specifically, there is a need for an occlusion detector that helps reduce the environmental footprint while still efficiently detecting and alerting the user when an occlusion occurs.

In this context, an aspect of the invention is an occlusion detector configured to detect occlusion during injection of a medical product into an injection site, the occlusion detector including:
a fluid chamber for allowing circulation of the medical product through the occlusion detector,
a piston rod configured for axially moving from an inactive position to an active position when a pressure exerted by the medical product in the fluid chamber increases,
a mechanical indicator configured for alerting a user when the piston rod reaches the active position.

The occlusion detector of the invention thus allows for detecting occlusion and alerting the user. The occlusion detector of the invention is more environmental friendly that occlusion detectors of the prior art, since the occlusion detector of the invention is purely mechanical. The occlusion detector of the invention does not include electronic components. The occlusion detector of the invention does not consume electrical power and is easier to recycle than electronic based occlusion detectors.

The device of the invention may further include some or all of the features listed below.

In an embodiment, the mechanical indicator includes a visual indicator that becomes visible to the user when the piston rod reaches the active position.

In an embodiment, the visual indicator includes a head portion of the piston rod.

In an embodiment, the occlusion detector includes a casing provided with an opening configured for receiving the head portion of the piston rod when the piston rod reaches the active position.

In an embodiment, the mechanical indicator includes a sound generator configured to emit a sound when the piston rod reaches the active position.

In an embodiment, the casing includes holes configured for allowing sound propagation outside the occlusion detector.

In an embodiment, the sound generator includes a resiliently deformable member connected to the housing.

In an embodiment, the resiliently deformable member is arranged on the axial path of the pison rod towards the active position such that the piston rod makes the resiliently deformable member swing when reaching the active position.

In an embodiment, the mechanical indicator includes a vibration generator configured to make the occlusion detector vibrate when the piston rod reaches the active position.

Therefore, the vibration generator acts as a shaker providing haptic feedback to the user.

In an embodiment, the vibration generator includes a resilient support and a hammer arranged on the resilient support for hitting a casing of the occlusion detector when the piston rod reaches the active position.

In an embodiment, the piston rod is separated from the fluid chamber by a resiliently deformable sealing membrane.

In an embodiment, the piston rod includes an actuator configured for axially abutting against the sound generator and/or the vibration generator when moving to the active position.

In an embodiment, the occlusion detector includes a spring for moving the piston rod back to the inactive position.

In an embodiment, the occlusion detector includes an inlet connector and an outlet connector for connecting the occlusion detector to an injection device.

Another aspect of the invention is an injection device including the above-described occlusion detector.

The invention and the advantages arising therefrom will clearly emerge from the detailed description that is given below with reference to the appended drawings as follows :
- Figures 1A, 1B are perspective views of an occlusion detector according to an embodiment of the invention,
- Figures 2A, 2B are cross-section views of an occlusion detector according to an embodiment of the invention, when no occlusion occurs,
- Figures 3A, 3B are perspective views of an occlusion detector according to an embodiment of the invention, when an occlusion is detected,
- Figure 3C is a cross-section view of an occlusion detector according to an embodiment of the invention, when an occlusion is detected,
- Figures 4A, 4B are, respectively, a perspective view and a cross-section view of an occlusion detector according to another embodiment of the invention, when no occlusion occurs,
- Figure 5A is a cross-section view of an occlusion detector according to an embodiment of the invention, when an occlusion is detected,
- Figures 5B, 5C are perspective views of an occlusion detector according to an embodiment of the invention, when an occlusion is detected,
- Figure 6A is a cross-section view of an occlusion detector according to an embodiment of the invention, when an occlusion is detected,
- Figure 6B illustrates different possible shapes of holes of an occlusion detector according to an embodiment of the invention,
- Figures 7A, 7B are perspective views of a wearable injection device of the injection, respectively when no occlusion occurs and when an occlusion is detected,
- Figures 8A, 8B are perspective views of a wearable injection device of the injection, respectively when no occlusion occurs and when an occlusion is detected,
- Figure 9A is a perspective view of an occlusion detector according to an embodiment of the invention,
- Figure 9B is a perspective view of an injection device including the occlusion detector of Figure 9A.

The different features of the embodiments can be used in combination with and used with other embodiments as long as the combined parts are not inconsistent with or interfere with the operation of the device and assembly. This invention is not limited in its application to the details of construction and the arrangement of components set forth in the following description or illustrated in the drawings. The embodiments herein are capable of being modified, practiced or carried out in various ways. The phraseology and terminology used herein is for the purpose of description and should not be regarded as limiting. The use of "including," "comprising," or "having" and variations thereof herein is to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not limited to physical or mechanical connections or couplings. In contrast, when an element is referred to as being "directly connected" or "directly coupled" to another element, there are no intervening elements present. Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein. Further, terms such as distal, proximal, up, down, bottom, and top are relative, and are to aid illustration, but are not limiting. Relative terms such as "below" or "above" or "upper" or "lower" or "horizontal" or "vertical" may be used herein to describe a relationship of one element to another element as illustrated in the Figures. It will be understood that these terms and those discussed above are intended to encompass different orientations of the device in addition to the orientation depicted in the Figures. The embodiments are not intended to be mutually exclusive so that the features of one embodiment can be combined with other embodiments as long as they do not contradict each other. Terms of degree, such as "substantially", "about" and "approximately" are understood by those skilled in the art to refer to reasonable ranges around and including the given value and ranges outside the given value, for example, general tolerances associated with manufacturing, assembly, and use of the embodiments. The term "substantially" when referring to a structure or characteristic includes the characteristic that is mostly or entirely present in the structure. As used herein, the singular forms "a," "an," and "the" are intended to include the plural forms as well, unless the context clearly indicates otherwise. As used herein, the term "and/or" includes any and all combinations of one or more of the associated listed items. It will be understood that, although the terms first, second, etc., may be used herein to describe various elements, these elements should not be limited by these terms. These terms are only used to distinguish one element from another. For example, a first element could be termed a second element, and, similarly, a second element could be termed a first element without departing from the scope of the present disclosure. For simplification, the parts or elements of one embodiment which are found identically or similarly in the other embodiment will be identified using the same numerical references and will not be described again.

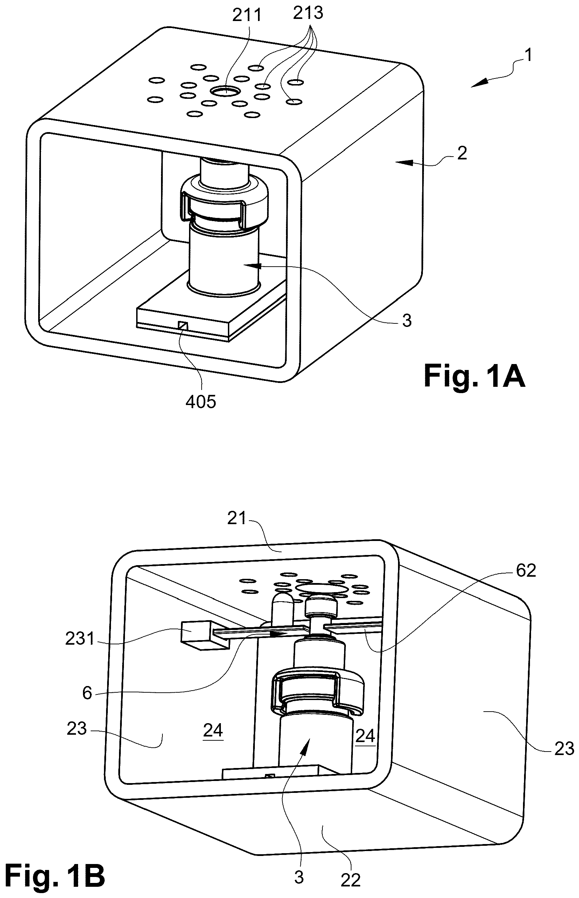

With reference to Figures 1A-1B is shown an occlusion detector 1 according to an embodiment of the invention. The occlusion detector 1 is configured for detecting when a partial or a full occlusion occurs in a fluid path of an injection device 100 during the injection process. The occlusion detector 1 includes some or all of a sensor 3 for detecting pressure generated by a medical product flowing from a reservoir, such as a syringe, a cartridge, a flexible bag or a pouch, to an injection site via a needle, a micro-needle or a catheter, a casing 2 for accommodating the sensor 3, and a mechanical indicator 6 for indicating to a user that an occlusion is detected.

By mechanical indicator 6 it is meant an indicator that does not include any electronic component. The mechanical thus does not require electrical power supply to indicate that occlusion is detected. The mechanical indicator 6 only includes mechanical components. The mechanical indicator 6 is devoid of any electronical or electrical circuitry.

With reference to Figures 1A-1B, the casing 2 is configured for containing the sensor 3 and the mechanical indicator 6. The casing 2 may have a cubic, parallelepipedic, or spheric shape ; the casing 2 may include an upper wall 21, a lower wall 22, and two or more side walls 23 delimiting an inner room for accommodating the sensor 3 and the mechanical indicator 6. The casing 2 may have two opposite opened sides 24. The casing 2 may be made of a single part, and may include or may be made of plastic material such as acrylonitrile-butadiene-styrene (ABS), methylmethacrylate acrylonitrile-butadiene-styrene (MABS), polycarbonate (PC), polyoxymethylene (POM), or any combination thereof.

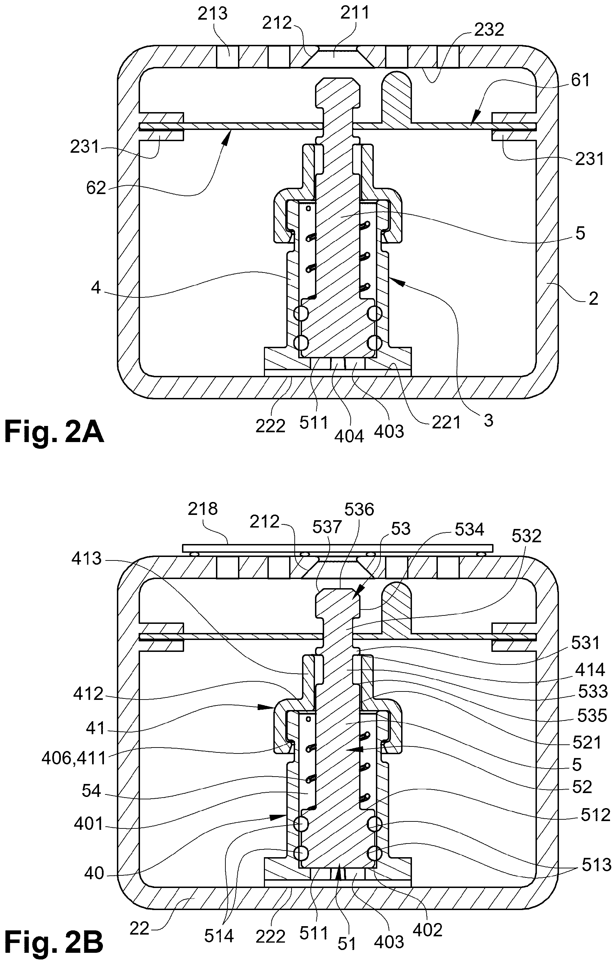

As illustrated in Figure 2A, the casing 2 may further include an opening 211 which may be arranged through the upper wall 21 for allowing a user to see that an occlusion is detected, one or more through-holes 213 which may be arranged through the upper wall 21 for allowing sound propagation outside the casing 2, a holder 231 for holding a sound generator 62 and/or a vibration generator 61, a striking surface 232 which may be arranged on the upper wall 21 for receiving impact of the vibration generator 61, a supporting surface 221 which may be defined by the lower wall 22 for supporting the sensor 3 and delimiting a fluid chamber 403 with a body of the sensor 3. The supporting surface 221 may include a film 222 for sealing the fluid chamber 403 and avoiding direct contact between the medical product circulating in the fluid chamber 403 and the lower wall 22 of the casing 2. The film 222 may include or may be made of a plastic material compatible with the drug in contact such as polycarbonate (PC) if the part is expected to be rigid or polyvinylidene fluoride (PVDF) if expected to be flexible.

As illustrated in Figure 2B, the wall which has the opening 211 and the holes 213, here the upper wall 21, may also include a membrane 218 configured for covering the opening 211 and the holes 213. The membrane 218 may be heat stacked or glued on the upper wall 21. The membrane 218 may be a see-through membrane 218, thereby allowing the user to see the visual indicator 536 when occlusion is detected. The membrane 218 may be flexible, and aims at providing a tight sealing when the injection device 100 or the casing 2 of the occlusion detector 1 needs to be tight.

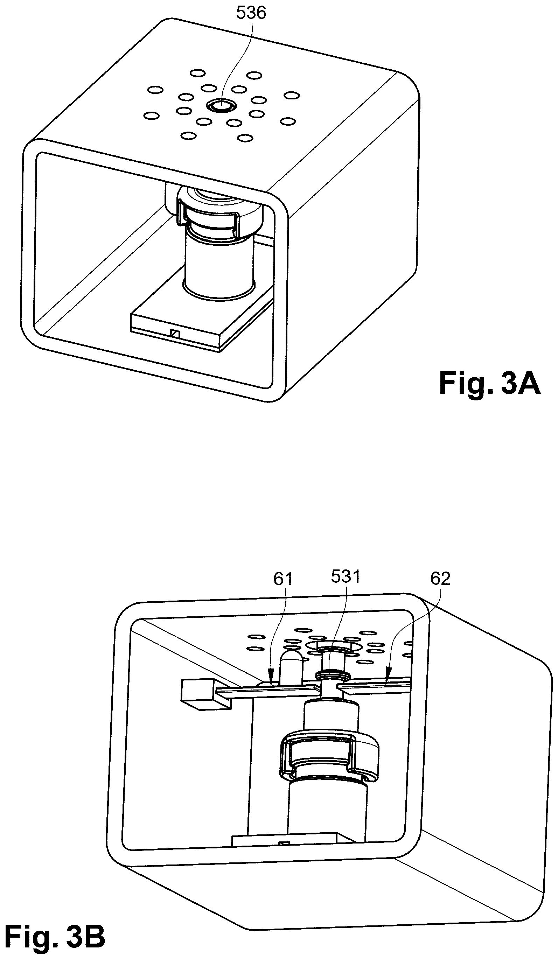

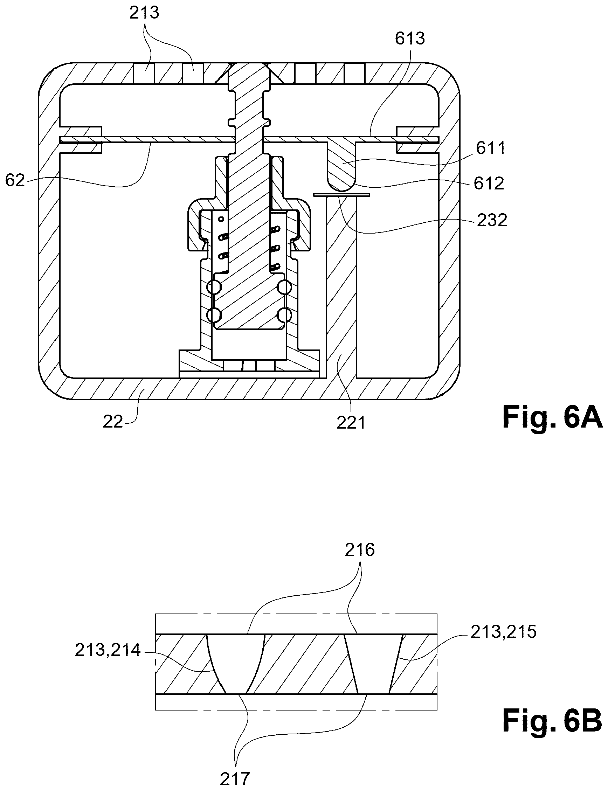

With reference to Figure 6B, the holes 213 allowing sound propagation may have different shapes. For instance, one or more holes 213 may have a paraboloid shape 214. In another example, one or more holes 213 may have a conical shape 215, or a tapered shape. Anyway, the holes 213 may preferably have an upper opening 216 larger than the lower opening 217, i.e. the lower opening 217 leading into the casing 2. In another embodiment, Figure, 6A, one or more holes 213 may have a cylindrical shape. The holes 213 may be regularly distributed in a circumferential direction around the opening 211, in one or more circular concentric rows, as shown for instance in Figures 3A, 5B or 8A. The shape and the arrangement of the holes 213 may help amplify the sound generated by the mechanical indicator 6.

With reference to Figure 2A, the sensor 3 is arranged within the casing 2 for reacting to the pressure of the medical product flowing towards the injection site.

The sensor 3 may be in the form of a cylinder including a housing 4 and a piston rod 5.

The housing 4 defines a fluid chamber 403 configured for being arranged in a fluid path of the medical product from the reservoir of the injection device 100 to the injection site. Therefore, when an occlusion occurs, pressure in the fluid chamber 403 increases. The body has an inlet aperture 404 for allowing entry of the medical product in the fluid chamber 403, the inlet aperture 404 being configured for being fluidly connected to an upstream portion of the fluid path, i.e. to the reservoir. The body further has an outlet aperture 405, Figure 1A, for allowing exit of the medical product outside the fluid chamber 403, the outlet aperture 405 being configured for being fluidly connected to a downstream portion of the fluid path leading to the injection site. The fluid chamber 403 may be arranged at a lower end of the body, and may be delimited by a lower body 40 of the housing 4, a lower sensing surface 511 of the piston rod 5, and either the film 222 or the supporting surface 221 of the casing 2.

As illustrated in Figure 2B, the housing 4 may include two parts, namely a lower body 40 and an upper body 41, assembled to each other by fasteners, such as snap-fit members 406, 411, clips, heat, adhesive, screw threads, etc. The lower body 40 includes a shoulder 402 for stopping the piston rod 5, the shoulder further separating the fluid chamber 403 from a sliding chamber 401 arranged above the fluid chamber 403 and configured for receiving the piston rod 5 and for allowing axial movement of the piston rod 5 within the lower body 40. The lower body 40 is fixed with respect to the casing 2. The film 222 and the lower body 40 of the sensor 3 may be made of a single piece.

The upper body 41 may include a seat 412, which may be a flat surface, for closing the sliding chamber 401 of the lower body 40 and for providing support to a spring 54 extending in the sliding chamber 401, around the piston rod 5, for biasing the piston rod 5 towards the fluid chamber 403, against the shoulder of the lower body 40. The upper body 41 may also include a guiding sleeve 413 arranged above the seat 412 for guiding axial movement of the piston rod 5 with respect to the upper body 41. The guiding sleeve 413 has a top opening 414 such that a portion of the piston rod 5 can extend outside the housing 4. The upper body 41 is fixed with respect to the lower body 40.

The housing 4 may be arranged at the center of the lower wall 22 of the casing 2 and may extend orthogonal to the supporting surface 221 of the casing 2, such that the piston rod 5 moves along an axis which is orthorgonal to the fluid path. The housing 4 is fixed with respect to the casing 2. Instead of being made of two different parts, and though not illustrated, the housing 4 could otherwise be made of a single piece.

Still with reference to Figure 2B, the piston rod 5 extends inside and outside the body and is configured for triggering the mechanical indicator 6 when the medical product pressure inside the fluid chamber 403 exceeds a predetermined threshold.

The piston rod 5 is in the form of an elongated rod extending along a vertical axis, which may include a lower portion 51 and a head portion 53 connected by a shaft portion 52. The piston rod 5 may be made of a single piece.

The lower portion 51 extends within the housing 4, and includes a preferably flat lower sensing surface 511 which faces and which may delimit the fluid chamber 403, a shoulder 512 opposite the lower sensing surface 511 for providing support to the spring 54, the shoulder 512 separating the lower portion 51 of the piston rod 5 from its shaft portion 52, and possibly one or more circumferential grooves 513 arranged on a lateral wall of the lower portion 51 for accommodating seals 514 such as O-ring seals ensuring dynamic sealing between the lower portion 51 of the piston rod 5 and an inner lateral surface of the lower body 40.

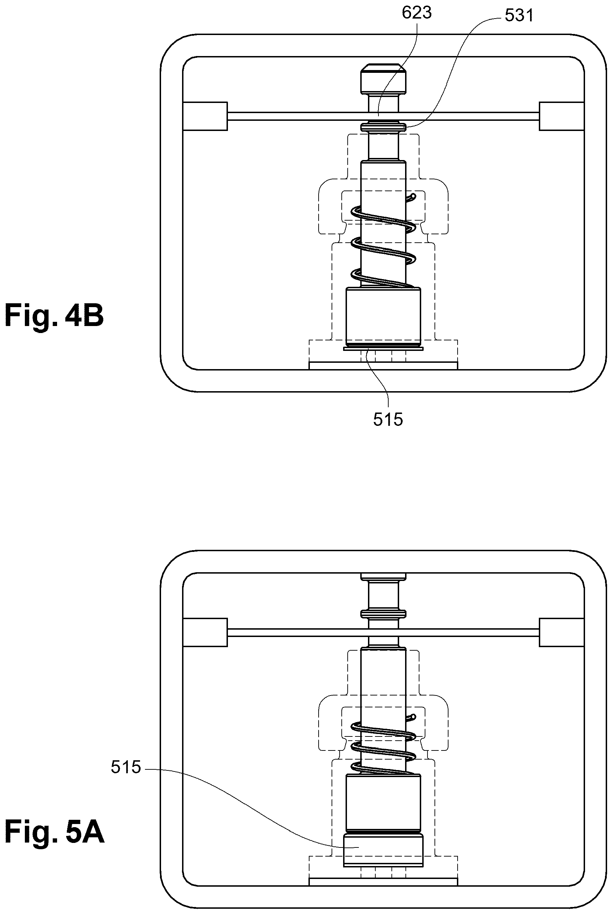

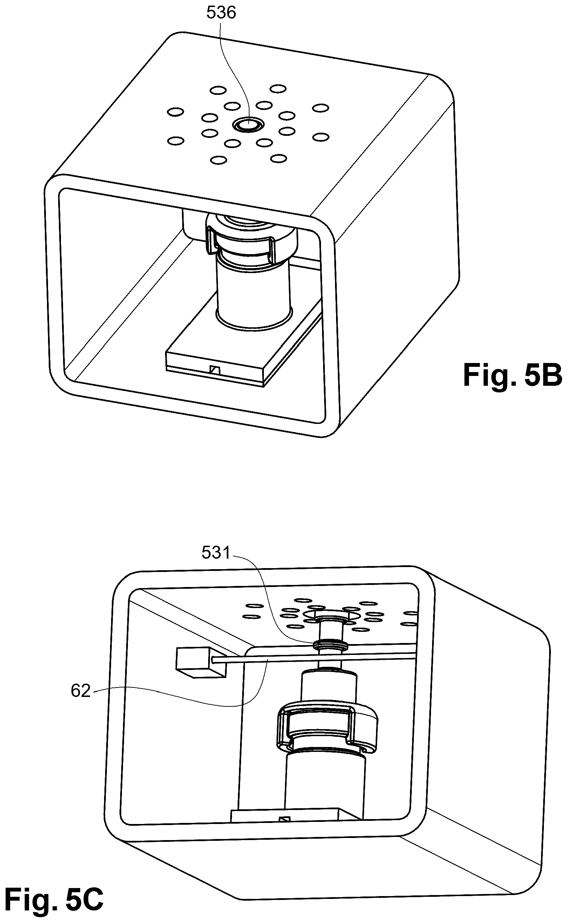

As illustrated in the Figures 4B and 5A, the O-ring seals may be replaced with a resiliently deformable sealing membrane 515 which is fixed to the lower body 40 of the sensor 3. This inflatable membrane 515 provides a static sealing, instead of a dynamic sealing, and avoids direct contact between the medical product and the piston rod 5. The membrane 515 can be flat or can be in the form of a flexible bellow.

Back to Figure 2B, the shaft portion 52 extends within the housing 4, and may have a lower diameter than the bottom portion and includes a lateral guiding surface 521 configured for cooperating with an inner lateral surface of the guiding sleeve 413 of the housing 4 for guiding axial movement of the piston rod 5.

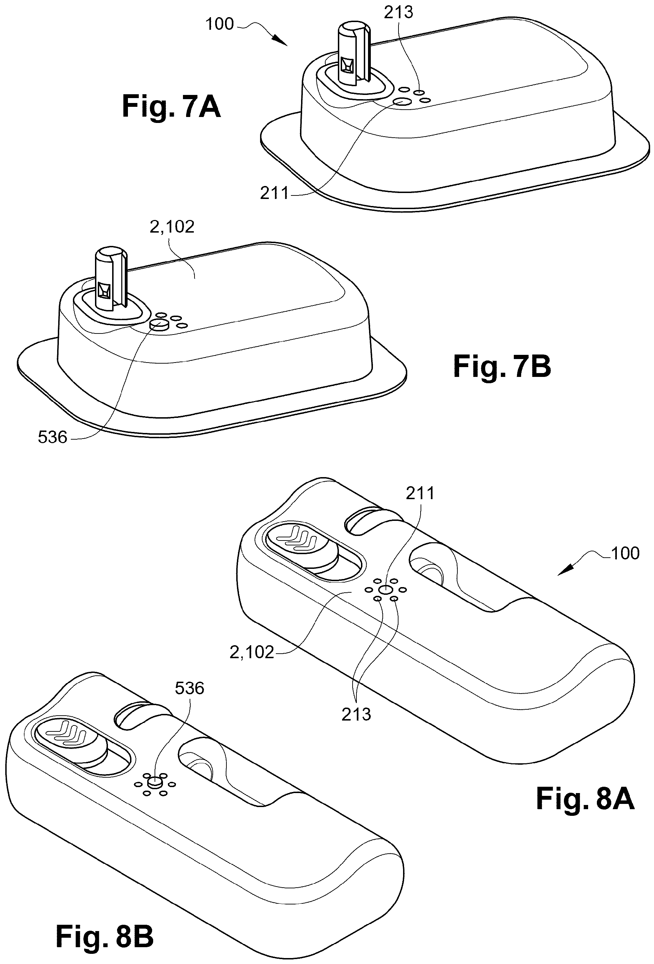

The head portion 53 partially or fully extends outside the housing 4 and may include a visual indicator 536, which may be an end surface arranged at the top end of the piston rod 5, the visual indicator 536 being configured for being concealed to the user as long as there is no occlusion and for becoming visible to the user when occlusion is detected. In the active position of the piston rod 5, the visual indicator 536 may either be flush with the upper wall 21 of the casing 2, see for instance Figure 6A, or may protrude beyond the upper wall 21 of the casing 2, see for instance Figures 7B, 8B.

The head portion 53 may further include a guiding surface 537, such as a chamfer, extending around the visual indicator 536 for guiding the visual indicator 536 towards the opening 211 of the casing 2 and cooperating with the tapered guiding surface 212 of the casing 2, thereby ensuring proper positioning of the visual indicator 536 when occlusion is detected. Between the visual indicator 536 and the shaft portion 52, the head portion 53 may include an actuator 531, such as a circumferential collar, configured for actuating other indicators such as a vibration generator 61, Figure 3B, configured for generating vibration of the casing 2 when occlusion happens and/or a sound generator 62 configured for generating a sound when occlusion happens. Above the actuator 531, the head portion 53 may have a first circumferential groove 532 defined between a first shoulder 534 and the actuator 531. Below the actuator 531, the head portion 53 may have a second circumferential groove 533 delimited by the actuator 531 and a second shoulder 535 which separates the head portion 53 from the shaft portion 52. The actuator 531 acts as a bump coming against the shaker 61 and the vibrating member 62 when the occlusion occurs.

The piston rod 5 is axially movable between an inactive position, Figures 2A-2B, in which no occlusion is detected such that the piston rod 5 does not activate the mechanical indicator 6, and an active position, Figures 3A-3C, in which occlusion is detected such that the piston activates the mechanical indicator 6 to inform the user that an occlusion is occuring. In the inactive position, the head portion 53 of the piston rod 5 may extend axially away from the casing 2, whereas in the active position, the head portion 53 of the piston rod 5 may bear against the casing 2. In the inactive position, the actuator 531 may be located beneath the sound generator 62, specifically between the sound generator 62 and the housing 4 of the sensor 3. In the active position, the actuator 531 may be located above the sound generator 62, specifically between the sound generator 62 and the opening 211 of the upper wall 21 of the casing 2.

Axial movement of the piston rod 5 between the inactive position and active position may be guided by the housing 4 of the sensor 3, and may be orthogonal to the fluid path and/or the supporting surface 221 of the casing 2, and may be caused by the pressure exerted by the medical product on the lower sensing surface 511 of the piston rod 5. When pressure increases, the piston rod 5 moves towards the active position. When pressure decreases, the piston rod 5 moves back towards the inactive position under the force exerted by the spring 54.

It is of note that the sensor 3 is a purely mechanical sensor 3 since occlusion is detected without use of any electronic component. The sensor 3 instead only includes mechanical parts and operates without need of electrical power supply.

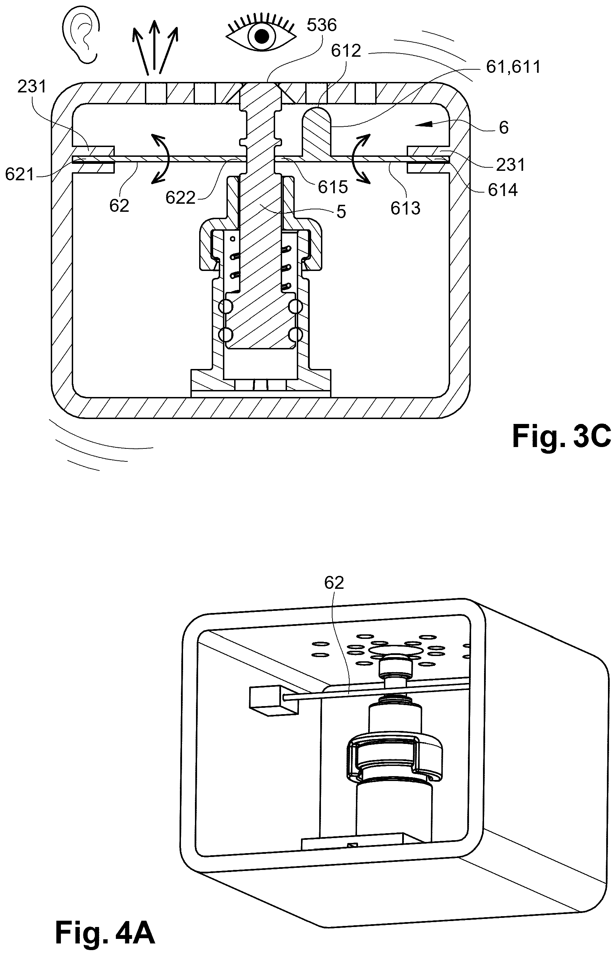

With reference to Figure 3C, the mechanical indicator 6 may include the visual indicator 536 of the piston rod 5, and/or the sound generator 62, and/or the vibration generator 61.

The sound generator 62 may be in the form of a resiliently deformable vibrating member, such as a metallic spring blade, which may include one end 621 fixed to the holder 231 of the casing 2 and one opposite free end 622 which may be arranged on the axial path of the actuator 531 such that movement of the piston rod 5 towards the active position causes the actuator 531 to axially abut against the free end 622 of the sound generator 62, causing deformation and then release of the sound generator 62, thereby generating a sound through the whole casing 2. The vibrations of the sound generator 62 provide the user with an audible feedback that an occlusion is occuring. The spring blade may extend orthogonal to the vertical movement axis of the piston rod 5.

In an alternative embodiment, illustrated in Figures 4A-4B and 5A-5C, the sound generator 62 may be in the form of a metallic wire having both ends fixed to the holders 231 of the casing 2. An intermediate portion 623, Figure 4B, of the metallic wire is arranged on the axial path of the actuator 531 such that movement of the piston rod 5 towards the active position causes the actuator 531 to axially abut against the intermediate of the sound generator 62, causing deformation and then release of the sound generator 62, thereby generating vibrations and sound through the whole casing 2. The metallic wire may extend orthogonal to the vertical movement axis of the piston rod 5. The mechanical indicator 6 may be devoid of the vibration generator 61.

Back to Figure 3C, the vibration generator 61 may include a hammer 611 configured for striking a striking surface of the casing 2 when occlusion is detected, thereby making the casing 2 vibrate when occlusion is detected. The vibration generator 61 thus acts as a shaker providing tactile indication to the user. The vibration generator 61 also produces some noise when the hammer 611 hits the casing 2, which also provides audible indication to the user. The hammer 611 may be arranged on a resilient support 613, which may be a spring blade, including a fixed end 614 secured to the holder 231 of the casing 2 and an opposite free end 615 which may be arranged on the axial path of the actuator 531 such that movement of the piston rod 5 towards the active position causes the actuator 531 to axially abut against the free end of the vibration generator 61, causing deformation and then release of the resilient support 613, thereby moving the hammer 611 up and down such that the hammer 611 strikes the casing 2 one or more times. The resilient support 613 may extend orthogonal to the vertical movement axis of the piston rod 5. The hammer 611 may extend orthogonal to the resilient support 613, closer to the free end 615 than to the fixed end 614. The hammer 611 may include a striking end 612 oriented in the upper direction, opposite the supporting surface 221.

In an alternative embodiment, Figure 6A, the hammer 611 may include a striking end 612 oriented in the lower direction, towards the supporting surface 221. The casing 2 may here include a post 221 extending for instance from the lower wall 22 of the casing 2, parallel to the piston rod 5, and having an opposite end defining the striking surface 232.

With reference to Figures 7A to 9B, the invention also relates to an injection device 100 including the above-described occlusion detector 1. The injection device 100 may be a wearable injection device 100 configured to be worn by the user, as shown in Figures 7A-7B or in Figures 8A-8B. The occlusion detector 1 may an add-on device arranged within the housing 102 of the injection device 100, or may be integrated in the housing 102 of the injection device 100, i.e. may be integral with the injection device 100. Specifically, the casing 2 of the occlusion detector 1 may also be the housing 102 of the injection device 100, as illustrated in Figures 7A to 8B.

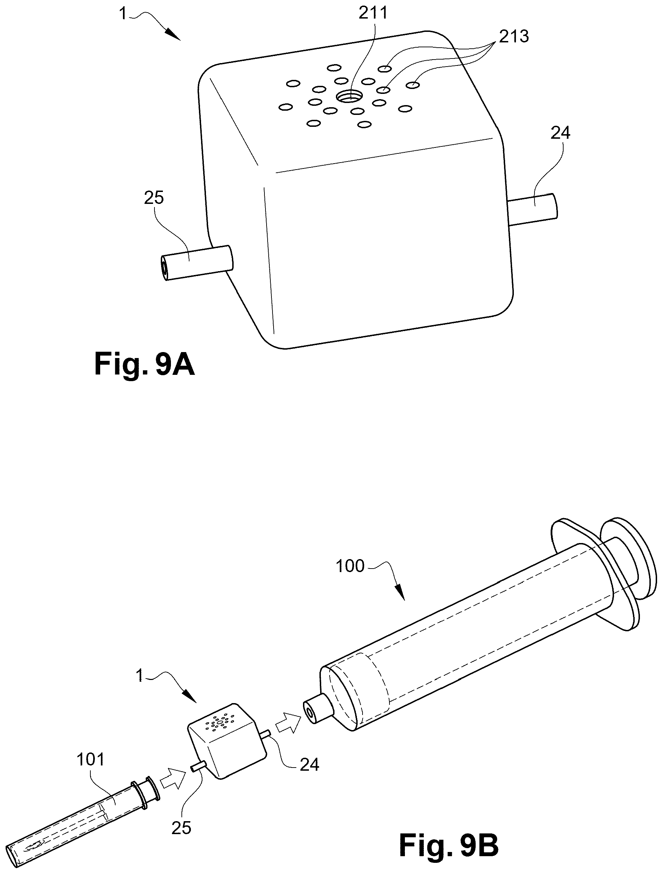

In another embodiment, Figures 9A-9B, the occlusion detector 1 is configured for being connected to an injection device 100, such as a syringe. To that end, the occlusion detector 1 may include an inlet connector 24, such as a luer, fluidly connected to the inlet aperture 404 of the fluid chamber 403 of the sensor 3, and an outlet connector 25, fluidly connected to the outlet aperture 405 of the fluid chamber 403 of the sensor 3. The inlet connector 24 may be for instance connected to the distal tip of the syring, as illustrated in Figure 9B, while the outlet connector 25 may be connected to a needle hub 101. The inlet connector 24 and/or the outlet connector 25 may be provided with a fastener, such as a thread, for allowing a luer lock connection or any standard connection means.

Operation of the occlusion detector 1 is described below.

When injection is being performed, the medical product flows through the fluid chamber 403 of the occlusion detector 1. If a partial or full occlusion happens, the pressure in the fluid chamber 403 increases. This causes the piston rod 5 to move up towards the active position. When moving up, the actuator 531 of the piston rod 5 bumps into the sound generator 62 and/or the support of the hammer 611. The sound generator 62 and/or the support vibrate. The sound generator 62 emits a sound. The hammer 611, hitting the casing 2, transmits vibrations to the casing 2, thereby providing haptic feedback that an occlusion is occuring. The support vibrations make the hammer 611 hit one or more times the casing 2. Meanwhile, the visual indicator 536, that is the free end of the piston rod 5, reaches the opening 211 of the casing 2, and may even extend therethrough, so that the user gets visual confirmation that an occlusion is taking place. The user can thus stop injection and/or serach the cause that led to the occlusion. If pressure is released, the spring 54 moves the piston rod 5 back towards the inactive position. The actuator 531 passes over the sound generator 62 and/or the support of the hammer 611, thereby providing feedback to the user that things are getting right again.

It is readily understandable from the above description that the occlusion detector 1 of the invention is purely mechanical and can provide the user with one, two or three different ways of indicating that an occlusion is detected. The occlusion detector 1 of the invention thus offers a reliable detection of occlusions with an improved sustainability. It is to be understood that the present invention is not limited to the embodiments described above and illustrated in the drawings; rather, the skilled person will recognize that many changes and modifications may be made within the scope of the appended claims.

For instance, the sensor 3 may be devoid of any spring 54. In such a case, the injection device 100 may stop when occlusion is detected and may not be restarted. The piston rod 5 here stays in the active position and does not move back to the inactive position.

## Claims

1. Occlusion detector (1) configured to detect occlusion during injection of a medical product into an injection site, the occlusion detector (1) including:
a fluid chamber (403) for allowing circulation of the medical product through the occlusion detector (1),
a piston rod (5) configured for axially moving from an inactive position to an active position when a pressure exerted by the medical product in the fluid chamber (403) increases,
a mechanical indicator (6) configured for alerting a user when the piston rod (5) reaches the active position.

2. Occlusion detector (1) according to the preceding claim, wherein the mechanical indicator (6) includes a visual indicator (536) that becomes visible to the user when the piston rod (5) reaches the active position.

3. Occlusion detector (1) according to the preceding claim, wherein the visual indicator (536) includes a head portion (53) of the piston rod (5).

4. Occlusion detector (1) according to the preceding claim, wherein the occlusion detector (1) includes a casing (2) provided with an opening (211) configured for receiving the head portion (53) of the piston rod (5) when the piston rod (5) reaches the active position.

5. Occlusion detector (1) according to any one of the preceding claims, wherein the mechanical indicator (6) includes a sound generator (62) configured to emit a sound when the piston rod (5) reaches the active position.

6. Occlusion detector (1) according to the preceding claim, wherein the casing (2) includes holes (213) configured for allowing sound propagation outside the occlusion detector (1).

7. Occlusion detector (1) according to any one of the preceding claims 5 or 6, wherein the sound generator (62) includes a resiliently deformable member connected to the housing (4).

8. Occlusion detector (1) according to the preceding claim, wherein the resiliently deformable member is arranged on the axial path of the piston rod (5) towards the active position such that the piston rod (5) makes the resiliently deformable member swing when reaching the active position.

9. Occlusion detector (1) according to any one of the preceding claims, wherein the mechanical indicator (6) includes a vibration generator (61) configured to make the occlusion detector (1) vibrate when the piston rod (5) reaches the active position.

10. Occlusion detector (1) according to the preceding claim, wherein the vibration generator (61) includes a resilient support (613) and a hammer (611) arranged on the resilient support (613) for hitting a casing (2) of the occlusion detector (1) when the piston rod (5) reaches the active position.

11. Occlusion detector (1) according to any one of the preceding claims, wherein the piston rod (5) is separated from the fluid chamber (403) by a resiliently deformable sealing membrane (515).

12. Occlusion detector (1) according to any one of the preceding claims 5-11, wherein the piston rod (5) includes an actuator (531) configured for axially abutting against the vibration generator (61) and/or the sound generator (62) when moving to the active position.

13. Occlusion detector (1) according to any one of the preceding claims, wherein the occlusion detector (1) includes a spring (54) for moving the piston rod (5) back to the inactive position.

14. Occlusion detector (1) according to any one of the preceding claims, wherein the occlusion detector (1) includes an inlet connector (24) and an outlet connector (25) for connecting the occlusion detector (1) to an injection device (100).

15. Injection device (100) including an occlusion detector (1) according to any one of the preceding claims.
